(19) Europäisches Patentamt
European Patent Office
Office européen des brevets

(11) Veröffentlichungsnummer: **0 327 489 B1**

(12) **EUROPÄISCHE PATENTSCHRIFT**

(45) Veröffentlichungstag der Patentschrift: **15.02.95**

(51) Int. Cl.$^{6}$: **C07J 1/00**, C07J 41/00

(21) Anmeldenummer: **89730020.8**

(22) Anmeldetag: **31.01.89**

# (54) Verfahren zur Herstellung von 14beta-Hydroxysteroiden.

(30) Priorität: **03.02.88 DE 3803551**

(43) Veröffentlichungstag der Anmeldung:
**09.08.89 Patentblatt 89/32**

(45) Bekanntmachung des Hinweises auf die Patenterteilung:
**15.02.95 Patentblatt 95/07**

(84) Benannte Vertragsstaaten:
**AT BE CH DE ES FR GB GR IT LI LU NL SE**

(56) Entgegenhaltungen:
**EP-A- 0 071 460**

(73) Patentinhaber: **SCHERING AKTIENGESELLSCHAFT**
**D-13342 Berlin (DE)**

(72) Erfinder: **Kirsch, Gerald, Dr.**
**Luciusstrasse 6b**
**D-1000 Berlin 33 (DE)**
Erfinder: **Golde, Roland**
**Dannenwalder Weg 174**
**D-1000 Berlin 26 (DE)**
Erfinder: **Neef, Günter, Dr.**
**Markgraf-Albrecht-Strasse 4**
**D-1000 Berlin 31 (DE)**

EP 0 327 489 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von 14β-Hydroxysteroiden der allgemeinen Formel I

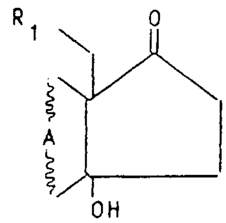

(I),

worin

- $R_1$ ein Wasserstoffatom oder eine Methylgruppe darstellt und
- A den Rest eines Steroides der Antrostan oder Estranreihe, welches im A, B, C-Ringsystem gesättigt ist, in diesem Ringsytem eine isolierte Doppelbindung besitzt oder im A-Ring aromatisch ungesättigt ist, symbolisiert.

14β-Hydroxysteroide, wie zum Beispiel die Herzglycoside sind pharmakologisch wirksame Substanzen (Angew. Chem., 63, 1951, 412 f; Arch. Pharmacol. 329, 1985, 414 f; J. Med. Chem. 30, 1987 1502 f; Biochem. Pharmacol., 30, 1981, 3001 f), ihre Synthese ist aber sehr aufwendig und oft technisch kaum durchführbar. So wird beispielsweise in der EP-A 071 460 ein Verfahren zur Herstellung zur Herstellung von 14β-Hydroxysteroiden beschrieben, bei dem man $\Delta^{14}$-Steroide mit N-Bromsuccinimid umsetzt und dann aus dem gebildeten Bromhydrin Bromwasserstoff abgespaltet. Dieses Verfahren ist aber abgesehen davon, daß es recht aufwendig ist auf ungesättigte Steroide nicht anwendbar.

Es wurde nun gefunden, daß man diese 14β-Hydroxysteroide wesentlich einfacher mittels eines Verfahrens synthestisieren kann, welches dadurch gekennzeichnet ist, daß man ein Steroid der allgemeinen Formel II

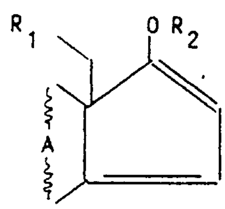

(II),

worin

- $R_1$ und A die oben genannte Bedeutung besitzen und
- $R_2$ einen 1-Oxoalkylrest mit 2 bis 6 Kohlenstoffatomen oder eine Benzoylgruppe bedeutet, mit einer Nitrosoverbindung der Formel III

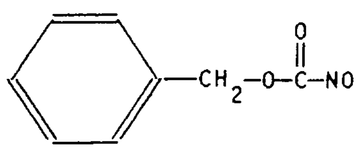

(III),

umsetzt,

das erhaltene Produkt in Gegenwart eines 1 bis 4 Kohlenstoffatome enthaltenden Alkohols erhitzt und die so

dargestellte Verbindung der allgemeinen Formel IV

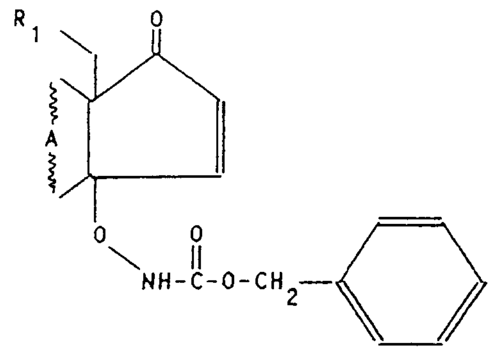

(IV),

worin

$R_1$ und A die oben genannte Bedeutung besitzen partiell hydriert.

Bei den, für das erfindungsgemäße Verfahren benötigten Ausgangsverbindungen der allgemeinen Formel II, handelt es sich um Steroide der Androstan- oder Estran-Reihe. Diese sind im A,B,C-Ringsystem gesättigt (5α H- und 5β H-Steroide) oder enthalten in diesem Ringsystem isolierte Doppelbindungen (beispielsweise in der 5(6)-, 7(8)-, 8(9)- oder 9(11)-Position) oder der A-Ring ist aromatisch ungesättigt. Meist tragen diese Steroide noch zusätzliche Substituenten wie ketalisierte Oxogruppen (wie zum Beispiel 2 bis 6 Kohlenstoffatome enthaltende Alkylendioxygruppen oder 1,2-Phenylendioxygruppen zum Beispiel in der 3-, 11-oder 12-Position), veresterte Hydroxygruppen (beispielsweise 1-Oxoalkyloxygruppen mit 2 bis 6 Kohlenstoffatomen oder Benzoyloxygruppen, zum Beispiel in der 3α-, 3β-, 11α-, 11β-, 12α oder 12β-Position) veretherte Hydroxygruppen (wie zum Beispiel die tert.-Butyloxygruppe oder die Benzyloxygruppe in der 3-, 11- oder 12-Position) oder Methylgruppen (beispielsweise in der 6- oder 7-Position).

In Hinblick auf die gewerbliche Verwertbarkeit der Verfahrensprodukte des erfindungsgemäßen Verfahrens sind solche Ausgangsverbindungen der allgemeinen Formel II bevorzugt, die die in dem Patentanspruch 2 gekennzeichneten Reste des Steroidmoleküls tragen.

Als 1-Oxoalkylgruppen $R_2$ und $R_3$ der Ausgangsverbindungen der allgemeinen Formel II seien beispielsweise genannt: die 1-Oxopropylgruppe, die 1-Oxo-2-methylpropylgruppe, die 2,2-Dimethyl-1-oxopropylgruppe, die 1-Oxobutylgruppe und insbesondere auch die Acetylgruppe.

Als Alkylgruppen $R_3$ sind beispielsweise geeeignet, die Methylgruppe, die Ethylgruppe, die Propylgruppe, die Isopropylgruppe oder die tert.-Butylgruppe.

Als Alkylendioxygruppen $R_4$ und $R_5$ dieser Ausgangssubstanzen eignen sich beispielsweise die Ethylendioxygruppe, die 1,3-Propylendioxygruppe oder die 2,2-Dimethyl-1,3-propylendioxygruppe.

Die erste Stufe des erfindungsgemäßen Verfahren wird in inerten Losungsmitteln, wie zum Beispiel chlorierten Kohlenwasserstoffen, wie Dichlormethan, Trichlormethan, 1,1,2,2-Tetrachlorethan, Ethern, wie Diethylether, Diisopropylether, 1,2-Dimethoxyethan, Dioxan oder Tetrahydrofuran oder aromatischen Kohlenwasserstoffen, wie Benzol oder Toluol durchgeführt.

Wegen der Instabilität der Nitrosoverbindung der Formel III ist es zweckmäßig, diese in der Reaktionsmischung durch Oxidation von Hydroxycarbaminsäure-phenylmethylester herzustellen.

Im Rahmen der vorliegenden Erfindung wurden bei den bislang durchgeführten Versuchen entsprechend der üblichen Praxis (J. Chem. Soc.; Chem. Comm. 1983, 1049 f; J. Chem. Soc. Perkin Trans. I, 1985, 1437f; Tetrahedron 40, 1984, 3695f) Tetraalkylammoniumperiodate mit 2 bis 6 Kohlenstoffatomen, wie das Tetrabutylammoniumperiodat, das Tetrapropylammoniumperiodat oder das Tetraethylammoniumperiodat) als Oxidationsmittel verwendet. Es ist aber für den Fachmann offensichtlich, daß diese Oxidation auch unter Verwendung anderer Oxidationsmittel durchgeführt werden kann. Zweckmäßigerweise wird das Oxidationsmittel der Reaktionsmischung in kleinen Anteilen über einen Zeitraum von etwa 10 Minuten bei 6 Stunden hinzugesetzt, um unerwünschte Nebenreaktionen zu vermeiden. Auch sollte ein Überschuß des Oxidationsmittels vermieden werden.

Die erste Stufe des erfindungsgemäßen Verfahren wird zweckmäßigerweise bei einer Reaktionstemperatur von - 30° C bis +20° C durchgeführt.

Das nach diesem ersten Reaktionsschritt erhaltene Reaktionsprodukt ist meist ein Gemisch von diastereoisomeren Steroiden. Es wird zweckmäßigerweise ohne weitere Reinigung in einem niederen Alkohol (mit vorzugsweise 1 bis 4 Kohlenstoffatomen, wie Methanol, Ethanol, Propanol, Isopropanol oder

Butanol) 15 Minuten bis 8 Stunden lang unter Rückfluß erhitzt (Reaktionstemperatur 65° C bis 130° C je nach verwendeten Alkohol) und man erhalt nach üblicher Aufbereitung sterisch reine Verbindungen der allgemeinen Formel IV. Andererseits ist es aber in der Regel nicht nicht erforderlich, die Reaktionsmischung aufzubereiten, sondern man kann diese Lösung direkt zur weiteren Umsetzung verwenden.

In dieser Reaktionsstufe kann die Reaktionsgeschwindigkeit durch Zugabe von katalytisch wirksamen Mengen einer Protonen- oder Lewis-Säure (wie zum Beispiel Chlorwasserstoff, Perchlorsaure, p-Toluolsulfonsäure oder Bortrifluorid) erhöht werden.

In der letzten Stufe des erfindungsgemäßen Verfahrens werden die Verbindungen der allgemeinen Formel IV partiell hydriert. Diese Hydrierung kann unter den Bedingungen durchgeführt werden, die dem Fachmann wohl bekannt sind (Houben Weyl "Methoden der Organischen Chemie" 4. Auflage, Band IV/1c Reduktion Teil 1, Georg Thieme Verlag Stuttgart, New York, 1980, 161 ff). Eine geeignete Methode ist beispielsweise die katalytische Hydrierung der Verbindungen in Gegenwart von Palladium-Katalysatoren. Diese Hydrierung kann in einem der oben genannten niederen Alkohole durchgeführt werden, man kann diese Substanzen aber auch in anderen Lösungsmitteln wie Ethylacetat, Dioxan, Tetrahydrofuran oder 1,2-Dimethoxyethan oder aber auch unter Verwendung anderer Katalysatoren wie Raney-Nickel partiell hydrieren.

Einige der erfindungsgemäß hergestellten Substanzen besitzen eine pharmakologische Wirksamkeit. Die meisten Verfahrensprodukte sind aber nur wertvolle Zwischenprodukte, die als Ausgangssubstanzen für die Synthese pharmakologisch wirksamer Steroide verwendet werden können. (Tetrahedron 22, 1966, 3189 f; Canad. J. Chem. 59, 1981 1403 f u.a.)

Die nachfolgenden Ausführungsbeispiele dienen zur näheren Erläuterung des erfindungsgemäßen Verfahrens.

## Beispiel 1

a) 3β-Acetoxy-14β-(benzyloxy-carbonylamino-oxy)-5,15-androstadien-17-on

Zur Lösung von 5,0 g 3β, 17-Diacetoxy-androsta-5,14,16-trien (hergestellt nach J. Pataki et al., J.Org.Chem. 37, 2137 (1972) in 30 ml Dichlormethan werden bei -15° C zunächst 3,0 g Hydroxycarbaminsaure-phenylmethylester zugesetzt und danach innerhalb von 60 Minuten tropfenweise eine Lösung von 7,78 g Tetrabutylammoniumperiodat in 30 ml Dichlormethan. Man läßt die Reaktionslosung innerhalb von 2 Stunden auf 0° C kommen, verdünnt dann mit Ethylacetat und wäscht sukzessive mit 15 % wässrige Natriumhydrogensulfit-Lösung, wässrige Natriumhydrogencarbonat-Lösung und Wasser. Nach Trocknung (Natriumsulfat) und Einengen erhält man 7,65 g eines öligen Rohprodukts, das ohne weitere Reinigung in 50 ml Methanol 4 Stunden unter Rückfluß erhitzt wird. Anschließend engt man das Volumen der Reaktionslösung auf ca. 30 ml ein und beläßt bei 0 bis -5° C zur Kristallisation. Nach dem Absaugen erhält man 3,0 g (45 % der Theorie) farblose Kristalle des Produkts vom Schmelzpunkt 182° C, $[\alpha]_D^{20}$ -51° (Chloroform, c=0,1).

b) 3β-Acetoxy-14β-hydroxy-5-androsten-17-on

Eine Losung von 3,4 g des nach Beispiel 1 a) erhaltenen Produkts in 158 ml Ethanol wird in Gegenwart von 648 mg Palladiumkohle (10 %) bei Raumtemperatur und Normaldruck hydriert. Nach Abfiltrieren des Katalysators und Einengen des Filtrats erhält man 2,75 g des Produkts. Nach Umkristallisation einer analytischen Probe erhält man farblose Nadeln von 3β-Acetoxy-14β-hydroxy-5-androsten-17-on vom Schmelzpunkt 228° C, $[\alpha]_D^{20}$ -23° (Chloroform, c=0,1). (Lit. P.J. Sykes et al., J.Chem. Soc. (C) 2346(1968) Schmelzpunkt 223-225° C $[\alpha]_D^{20}$ -20°, (Chloroform, c=0,1).

## Beispiel 2

a) 14β-(Benzyloxy-carbonylamino-oxy)-3-methoxy-estra-1,3,5(10),15-tetraen-17-on

Unter den Bedingungen des Beispiels 1 a) setzt man 4,8 g 17-Acetoxy-3-methoxy-estra-1,3,5(10),14,16-pentaen (Herstellung nach G.H. Rasmussen et al., Steroids 22, 107 (1973) in 25 ml Dichlormethan mit 2,57 g Hydroxycarbaminsäure-phenylmethylester und 6,67 g Tetrabutylammoniumperiodat in 26 ml Dichlormethan um. Man erhält 5,28 g (80 %) des Produkts vom Schmelzpunkt 178-179° C, $[\alpha]_D^{20}$ +86,6° (Chloroform, c=0,51).

b) 14β-Hydroxy-3-methoxy-estra-1,3,5(10)-trien-17-on

Eine Lösung von 1,51 g des unter Beispiel 2 a) erhaltenen Produkts in 75 ml Ethanol wird unter den Bedingungen des Beispiels 1 b) hydriert. Nach Umkristallisation des Rohprodukts aus Dichlormethan/Diisopropylether erhält man 930 mg (92 %) 14β-Hydroxy-3-methoxy-estra-1,3,5(10)-trien-17-on vom Schmelzpunkt 173-175° C, $[\alpha]_D^{20}$ +103,4° (Chloroform, c=0,525). (Lit. G. Schubert und K. Ponsold, Pharmazie 34, 323 (1979) Schmelzpunkt 172-175° C, $[\alpha]_D^{20}$ +105°).

**Patentansprüche**

**1.** Verfahren zur Herstellung von 14β-Hydroxysteroiden der allgemeinen Formel I

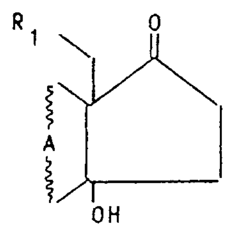

(I),

worin

R₁ ein Wasserstoffatom oder eine Methylgruppe darstellt und

A den Rest eines Steroides der Antrostan oder Estranreihe, welches im A, B, C-Ringsystem gesättigt ist, in diesem Ringsytem eine isolierte Doppelbindung besitzt oder im A-Ring aromatisch ungesättigt ist, symbolisiert, dadurch gekennzeichnet, daß man ein Steroid der allgemeinen Formel II

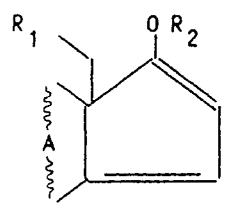

(II),

worin

$R_1$ und A die oben genannte Bedeutung besitzen und

$R_2$ einen 1-Oxoalkylrest mit 2 bis 6 Kohlenstoffatomen oder eine Benzoylgruppe bedeutet, mit einer Nitrosoverbindung der Formel III

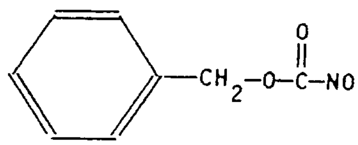

(III),

umsetzt,

das erhaltene Produkt in Gegenwart eines 1 bis 4 Kohlenstoffatome enthaltenden Alkohols erhitzt und die so dargestellte Verbindung der allgemeinen Formel IV

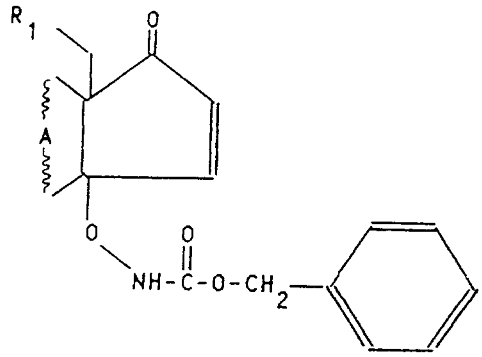

(IV),

worin

$R_1$ und A die oben genannte Bedeutung besitzen partiell hydriert.

**2.** Verfahren zur Herstellung von 14β-Hydroxysteroiden der allgemeinen Formel I gemäß Patentanspruch 1, dadurch gekennzeichnet, daß der Rest des Steroid-Moleküls A eine Gruppierung der Teilformel Va oder Vb

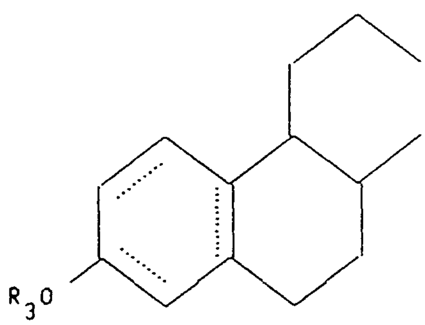

(Va),

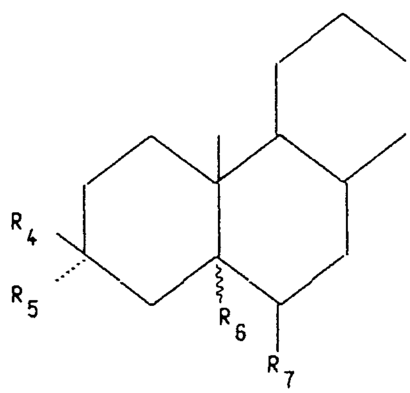

(Vb),

bedeuten, worin

die Bindungen ..... Einfachbindungen oder Doppelbindungen darstellen,

$R_3$ eine 1 bis 4 Kohlenstoffatome enthaltende Alkylgruppe, eine Benzylgruppe, eine 2 bis 6 Kohlenstoffatome enthaltende 1-Oxoalkylgruppe oder eine Benzoylgruppe bedeutet,

$R_4$ und $R_5$ gemeinsam eine 2 bis 6 Kohlenstoffatome enthaltende Alkylendioxygruppe oder eine 1,2-Phenylendioxygruppe symbolisieren oder $R_4$ die gleiche Bedeutung wie $OR_3$ besitzt und $R_5$ ein Wasserstoffatom darstellt und

$R_6$ und $R_7$ gemeinsam eine Kohlenstoff-Kohlenstoffbindung oder jeweils ein Wasserstoffatom bedeuten.

**3.** Verfahren zur Herstellung von 14β-Hydroxysteroiden der allgemeinen Formel I gemäß Patentanspruch 1 und 2, dadurch gekennzeichnet, daß man die zur Umsetzung benötigte Nitrosoverbindung der Formel III in der Reaktionsmischung durch Oxidation von Hydroxycarbaminsäure-phenylmethylester der Formel VI

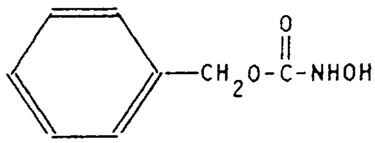

(VI),

herstellt.

**Claims**

**1.** Process for the preparation of 14β-hydroxy steroids of the general formula I

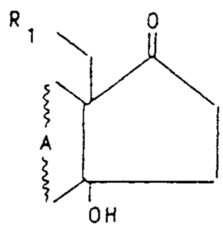

(I)

wherein
$R_1$ represents a hydrogen atom or a methyl group and A represents the residue of a steroid of the androstane or oestrane series, which is saturated in the A,B,C-ring system, possesses an isolated double bond in that ring system or is aromatically unsaturated in the A-ring, characterised in that a steroid of the general formula II

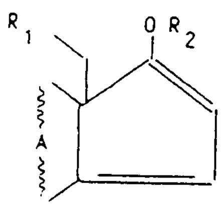

(II)

wherein $R_1$ and A are as defined above and $R_2$ represents a 1-oxoalkyl radical having from 2 to 6 carbon atoms or a benzoyl group,
is reacted with a nitroso compound of formula III

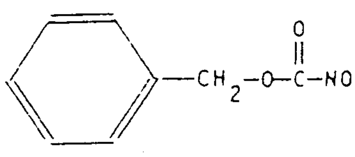

(III),

the resulting product is heated in the presence of an alcohol containing from 1 to 4 carbon atoms, and the resulting compound of the general formula IV

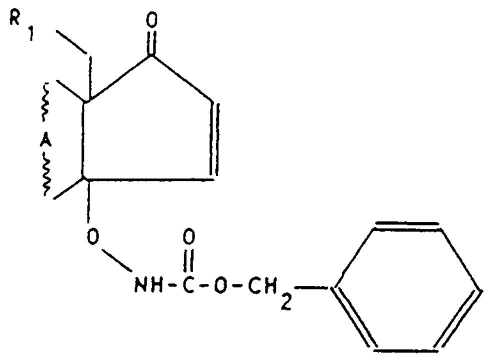

(IV),

in which
$R_1$ and A are as defined above, is partially hydrogenated.

**2.** Process for the preparation of 14$\beta$-hydroxy steroids of the general formula I according to patent claim 1, characterised in that the residue of the steroid molecule A represents a grouping of the partial formula Va or Vb

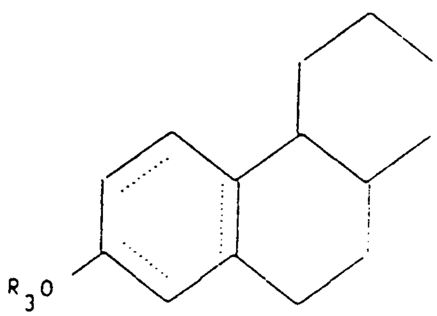

(Va)

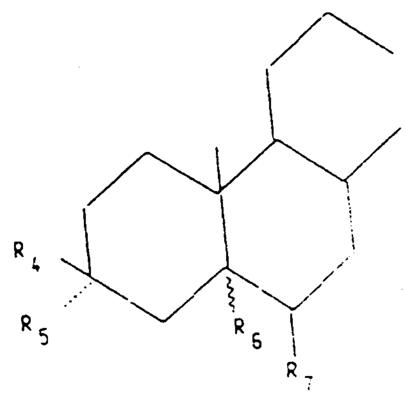

(Vb),

wherein
the bonds ..... represent single or double bonds, $R_3$ represents an alkyl group containing from 1 to 4 carbon atoms, a benzyl group, a 1-oxoalkyl group containing from 2 to 6 carbon atoms or a benzoyl group, $R_4$ and $R_5$ together represent an alkylenedioxy group containing from 2 to 6 carbon atoms or a 1,2-phenylenedioxy group, or $R_4$ has the same meaning as $OR_3$ and $R_5$ represents a hydrogen atom, and
$R_6$ and $R_7$ together represent a carbon-carbon bond or each represents a hydrogen atom.

**3.** Process for the preparation of 14$\beta$-hydroxy steroids of the general formula I according to either claim 1 or claim 2, characterised in that the nitroso compound of formula III required for the reaction is prepared in the reaction mixture by the oxidation of a hydroxycarbamic acid phenyl methyl ester of formula VI

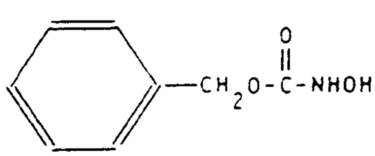

(VI).

## Revendications

1. Procédé pour la préparation de 14$\beta$-hydroxystéroïdes de formule générale I

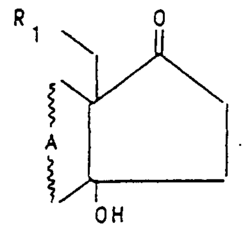

(I),

où
$R_1$ représente un atome d'hydrogène ou un groupe méthyle et
A symbolise le radical d'un stéroïde de la série de l'antrostane ou de l'estrane, qui est saturé dans le système de cycles A, B, C, qui a une double liaison isolée dans ce système de cycles ou est aromatiquement insaturé dans le cycle A, caractérisé en ce qu'on fait réagir un stéroïde de formule générale II

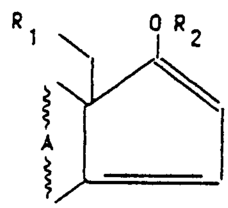

(II),

dans laquelle
$R_1$ et A ont la signification donnée ci-dessus et $R_2$ signifie un radical 1-oxoalkyle comportant de 2 à 6 atomes de carbone ou un groupe benzoyle, avec un composé nitroso de formule III

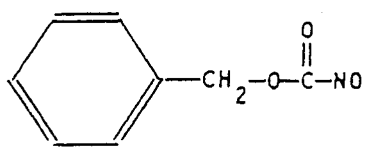

(III),

on chauffe le produit obtenu, en présence d'un alcool comportant de 1 à 4 atomes de carbone, et on hydrogène partiellement le composé ainsi formé de formule générale IV

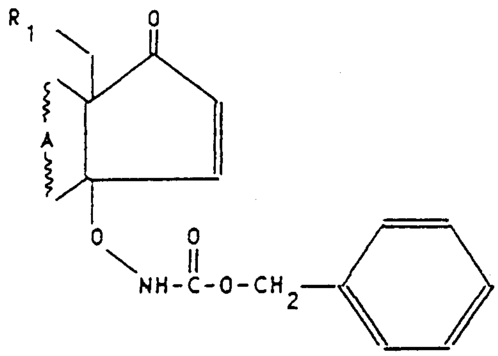

(IV),

dans laquelle
$R_1$ et A ont la signification donnée ci-dessus.

2. Procédé pour la préparation de 14β-hydroxystéroïdes de formule générale I, selon la revendication 1, caractérisé en ce que le radical de la molécule du stéroïde A signifie un groupement de formule partielle Va ou Vb

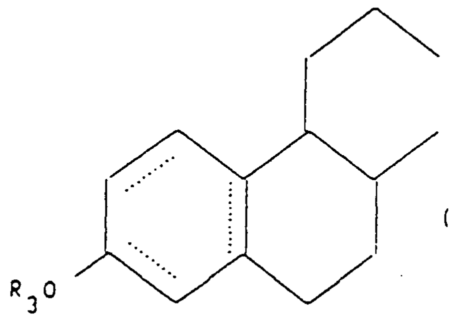

(Va),

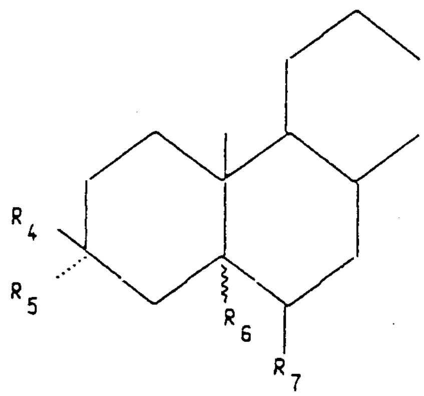

(Vb),

où
les liaisons ....... représentent des liaisons simples ou des liaisons doubles,

| | |
|---|---|
| $R_3$ | représente un groupe alkyle comportant de 1 à 4 atomes de carbone, un groupe benzyle, un groupe 1-oxoalkyle comportant de 2 à 6 atomes de carbone ou un groupe benzoyle, |
| $R_4$ et $R_5$ | ensemble symbolisent un groupe alkylènedioxy comportant de 2 à 6 atomes de carbone ou un groupe 1,2-phénylènedioxy, ou $R_4$ a la même signification que $OR_3$ et $R_5$ représente un atome d'hydrogène et |
| $R_6$ et $R_7$ | ensemble signifient une liaison carbone-carbone ou chaque fois un atome d'hydrogène. |

3. Procédé pour la préparation de 14β-hydroxystéroïdes de formule générale I selon les revendications 1 et 2, caractérisé en ce qu'on prépare le composé nitroso de formule III nécessaire à la réaction, dans le mélange réactionnel, par oxydation de l'ester phénylméthylique de l'acide hydroxycarbaminique de formule VI.

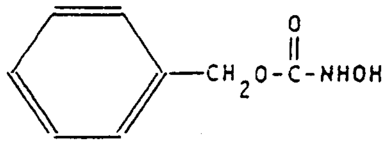

(VI),